# FASCICULE DE BREVET EUROPEEN

(11) **EP 2 035 064 B1**
(45) Date de publication et mention de la délivrance du brevet: **04.09.2013**
(21) Numéro de dépôt: 07823542.1
(22) Date de dépôt: 03.07.2007
(51) Int. Cl.: A61M 5/32

(54) **CAPUCHON D'AIGUILLE**
NADELKAPPE
NEEDLE CAP

(30) Priorité: 04.07.2006 FR 0652786
(43) Date de publication de la demande: 18.03.2009
(73) Titulaire: ALLERGAN INDUSTRIE, 74370 Pringy (FR)
(72) Inventeur: GANTIN, Denis, 74130 Ayse (FR); MANDAROUX, Bastien, 74960 Cran Gevrier (FR)
(74) Mandataire: Cardy, Sophie Marie
(86) Numéro de dépôt international: PCT/FR2007/051586
(87) Numéro de publication internationale: WO 2008/003899

(56) Documents cités:
- EP-A- 1 530 979
- FR-A1- 2 259 624

## Description

La présente invention a pour objet un capuchon d'aiguille pour seringue et une seringue équipée dudit capuchon.

De façon plus précise, la présente invention concerne un capuchon pour aiguille de seringue qui permet d'améliorer la fixation de l'aiguille sur le corps de seringue.

L'invention concerne plus particulièrement un type de seringue utilisé notamment mais non exclusivement dans le domaine de la médecine esthétique, dans la dermatologie et notamment pour l'injection sous-cutanée de produits aptes à remodeler l'aspect de la peau. Le document FR-A1-2259 624 décrit un capuchon d'aiguille tel que celui décrit dans le préambule de la revendication 1.

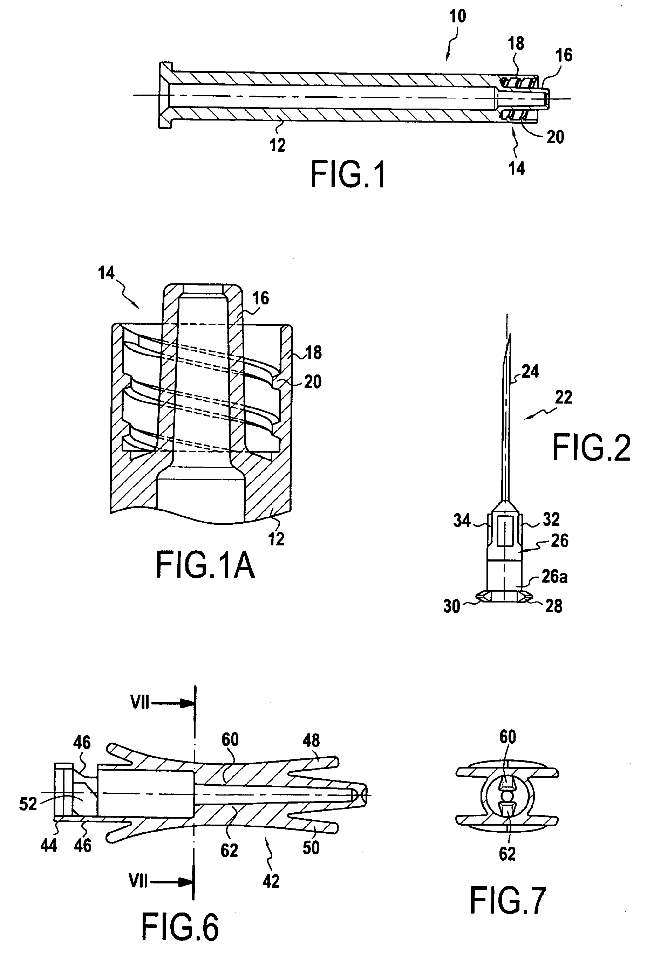

L'invention s'applique notamment à une seringue et a un ensemble aiguille du type représenté sur les figures 1, 1A et 2.

La seringue 10 représentée sur les figures 1 et 1A comprend de façon classique un corps cylindrique 12 présentant une extrémité de fixation 14. L'extrémité de fixation 14 est constituée par un tronc de cône de centrage 16 et par une virole 18 entourant le cône 16 et munie d'un taraudage 20.

Sur la figure 2, on a représenté l'ensemble aiguille 22 qui est constitué par l'aiguille proprement dite 24 et par une embase creuse 26. A son extrémité 26a opposée à l'aiguille, l'embase est munie de deux projections radiales 28 et 30 qui forment des saillies aptes à coopérer avec le taraudage 20 de la virole 18 du corps de seringue 12. Les deux projections radiales constituent une portion de filetage. A son autre extrémité, l'embase 26 comporte de préférence deux ailettes 32 et 34 dont la fonction sera explicitée ultérieurement.

Pour mettre en place l'ensemble aiguille 22 sur la seringue 10, on engage le passage interne de l'embase 26 sur l'extrémité du tronc de cône 16 et les extensions radiales 28 et 30 coopèrent avec le taraudage 20 du corps de seringue. En faisant tourner l'embase de l'aiguille, on obtient un vissage de l'aiguille sur le corps de seringue.

L'expérience montre que dans le cas où le liquide qui doit être injecté et relativement fluide, le mode de fixation de l'aiguille sur le corps de seringue est tout à fait satisfaisant. En revanche, lorsque le liquide à injecter présente une plus grande viscosité, ce mode de liaison s'avère souvent insuffisant surtout si lors du montage de l'aiguille sur le corps de seringue l'utilisateur ne visse pas suffisamment l'embase sur le corps de seringue. Une telle situation se rencontre notamment mais non exclusivement lorsqu'on veut injecter sous la peau du patient des composés à base d'acide hyaluronique dont la viscosité est relativement importante.

Un premier objet de la présente invention est de fournir un capuchon d'aiguille pour seringue, notamment du type décrit en liaison avec les figures 1, 1A et 2 qui non seulement protège l'aiguille mais permet également d'assurer une fixation très fiable de l'aiguille sur le corps de seringue.

Pour atteindre ce but selon l'invention, le capuchon pour aiguille de seringue destinée à être montée sur le corps d'une seringue se caractérise en ce qu'il comprend :
- une partie formant enveloppe pour recevoir l'aiguille ;
- une pièce de fixation apte à recevoir l'embase de l'aiguille et comportant sur sa face externe un filetage apte à coopérer avec un taraudage ménagé à une extrémité du corps de seringue ; et
- des portions sécables pour relier ladite partie formant enveloppe et ladite pièce de fixation, les portions sécables ayant des dimensions déterminées pour qu'elles se brisent lorsqu'un couple prédéterminé est appliqué à la partie formant enveloppe alors que le filetage de la pièce de fixation a été engagé avec le taraudage du corps de seringue.

On comprend que les portions sécables sont déterminées de telle manière qu'elles soient brisées lorsque le couple de serrage du capuchon sur le corps de seringue est suffisant. Ainsi, la pièce de fixation reste vissée dans le taraudage du corps de seringue alors que la partie formant enveloppe du capuchon est libérée. La pièce de fixation qui coopère avec le taraudage constitue l'équivalent d'un contre-écrou de blocage assurant ainsi une fixation très fiable de l'aiguille sur le corps de seringue.

De préférence, la partie formant enveloppe, la pièce de fixation et les portions sécables sont formées dans un même composant, les portions sécables étant constituées par des zones de dimensions réduites limitées par des orifices réalisés dans ledit composant.

De préférence encore, les orifices ont des contours tels qu'après section des portions sécables, la partie des portions sécables restant liée à la pièce de fixation forme des saillies aptes à coopérer en rotation autour de l'axe longitudinal du capuchon avec la partie des portions sécables restant liées à la partie formant enveloppe également en saillie pour permettre le dévissage de la portion de fixation par rapport au corps de la seringue.

On comprend que la périphérie des orifices qui définissent les portions sécables est définie de telle manière que, après séparation de la partie formant enveloppe par rapport à la pièce de fixation, les parties des portions sécables restant liées à la partie formant enveloppe peuvent être utilisées comme une sorte de tournevis, ces parties en saillie coopérant avec les parties des portions sécables restant liées à la pièce de fixation.

De préférence également, la face interne de la partie formant enveloppe comporte des reliefs aptes à coopérer en rotation avec des reliefs ménagés dans l'embase de l'aiguille, lorsque les parties en saillie des portions sécables restant liées à la pièce de fixation et à la partie formant enveloppe coopèrent en rotation.

On comprend que les reliefs réalisés dans la face interne de la partie formant enveloppe peuvent coopérer avec des reliefs correspondants ménagés dans l'embase de l'aiguille lorsque les parties en saillie des portions sécables restant liées à la pièce de fixation et la partie formant enveloppe coopèrent en rotation pour réaliser le dévissage de la pièce de fixation par rapport au corps de seringue. Cette rotation de la partie formant enveloppe permet simultanément le dévissage de l'aiguille par coopération des reliefs ménagés dans l'embase de l'aiguille et des reliefs ménagés dans la face interne de la partie formant enveloppe.

Un deuxième objet de l'invention est de fournir une seringue du type décrit précédemment et équipée d'un capuchon du type défini également précédemment.

Selon l'invention, la seringue comprend :
- un corps de seringue présentant une extrémité de fixation munie d'un taraudage ;
- un ensemble aiguille comprenant une aiguille et une embase, ladite embase comportant deux extensions radiales aptes à coopérer avec ledit taraudage du corps de seringue ; et
- un capuchon d'aiguille ;
   ladite seringue se caractérise en ce que ledit capuchon d'aiguille comprend :
- une partie formant enveloppe pour recevoir l'aiguille proprement dite ;
- une pièce de fixation apte à recevoir l'embase de l'aiguille et comportant sur sa face externe un filetage apte à coopérer avec le taraudage ménagé à l'extrémité du corps de seringue ; et
- des portions sécables pour relier ladite partie formant enveloppe et ladite pièce de fixation, les portions sécables ayant des dimensions déterminées pour qu'elles se brisent lorsqu'un couple prédéterminé est appliqué à la partie formant enveloppe alors que le filetage de la pièce de fixation a été engagé avec le taraudage du corps de seringue, la pièce de fixation formant alors dans le taraudage du corps de seringue un contre-écrou pour les extensions radiales de l'embase de l'ensemble aiguille également engagées dans le taraudage.

D'autres caractéristiques et avantages de l'invention apparaîtront mieux à la lecture de la description qui suit de modes préférés de réalisation de l'invention donnés à titre d'exemples non limitatifs. La description se réfère aux figures annexées, sur lesquelles :
- la figure 1 déjà décrite montre en coupe longitudinale un corps de seringue ;
- la figure 1A montre le détail de l'extrémité du corps de seringue sur lequel doit être fixée l'aiguille ;
- la figure 2 déjà décrite montre un ensemble aiguille utilisable dans l'invention ;
- la figure 3 est une vue de côté du capuchon pour aiguille ;
- la figure 4 est une vue de dessus du capuchon pour aiguille ;
- la figure 5 est une vue en coupe selon la ligne V-V de la figure 3 ;
- la figure 6 est une vue en coupe longitudinale selon la ligne VI-VI de la figure 4 ;
- la figure 7 est une vue en coupe transversale suivant la ligne VII-VII de la figure 6 ;
- la figure 8 est une vue en perspective de l'extrémité du capuchon d'aiguille ;
- la figure 9 est une vue en coupe partielle longitudinale montrant la fixation de l'aiguille et du capuchon sur le corps de seringue ; et
- la figure 9 A est une vue de détail selon la ligne A-A de la figure 9.

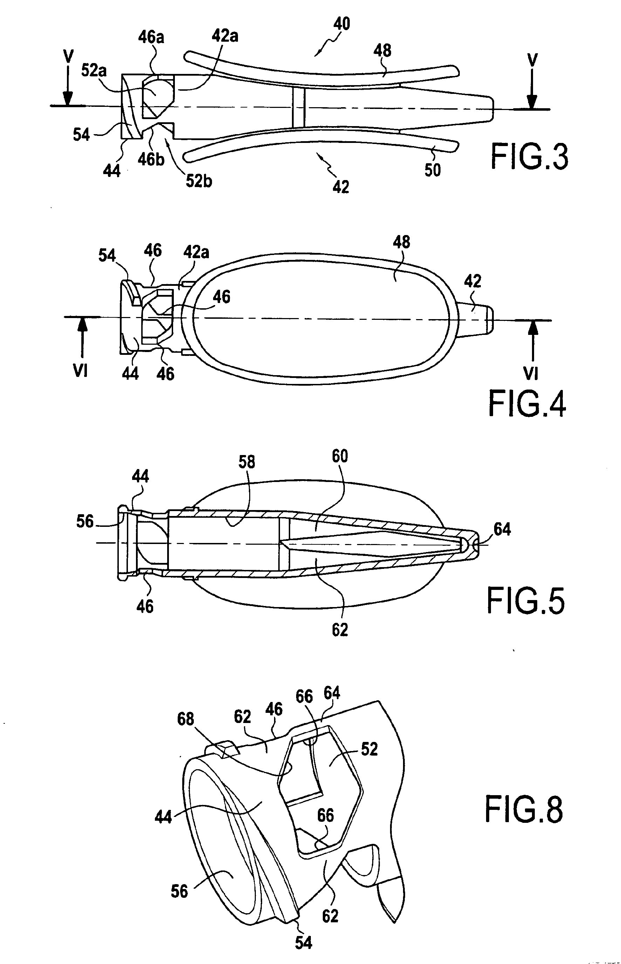

Comme le montre la figure 3, le capuchon 40 comprend une partie formant enveloppe 42, une pièce de fixation 44 sur le corps de seringue et des portions sécables telles que 46a, 46b qui relient l'extrémité 42a de la partie formant enveloppe à la pièce de fixation 44. La partie formant enveloppe 42 est équipée de préférence de deux surfaces externes de préhension 48 et 50.

De préférence, le capuchon est réalisé par moulage. Les différents éléments constitutifs du capuchon à savoir la partie formant enveloppe 42, la pièce de fixation 44 et les portions sécables 46a, 46b sont réalisés à partir d'un même ensemble. Les portions sécables 46a, 46b sont définies par des orifices tels que 52a, 52b. Ces orifices définissent également l'extrémité 42a de la partie formant enveloppe 42 et une des extrémités de la pièce de fixation 44. La pièce de fixation 44 est munie sur sa face externe d'un filetage 54 apte à coopérer avec le taraudage 20 de la virole 18 du corps de seringue. Comme le montre mieux la figure 5, la pièce de fixation 44 présente un passage axial 56 qui a une forme légèrement ovoïde en section droite pour coopérer avec l'embase 26 de l'ensemble aiguille 22. Le passage axial 56 assure un certain serrage de l'embase 26 de l'aiguille sans réaliser néanmoins une solidarisation efficace.

Sur la face interne 58 de la partie formant enveloppe, on réalise deux nervures en forme de relief 60 et 62 disposées entre la partie centrale de la partie formant enveloppe 42 et son extrémité fermée 64. Ces nervures internes 60 et 62 sont aptes à coopérer avec les ailettes 32 et 34 de l'embase 26 de l'ensemble aiguille comme on l'expliquera ultérieurement.

En se référant à la figure 8, on va décrire plus en détail la forme particulière du contour des orifices 52 qui définissent les portions sécables 46. Chaque portion sécable 46 comporte une partie 62 qui reste liée à la pièce de fixation 44 et une partie 64 qui reste liée à la partie formant enveloppe 42 après section des portions sécables. Les portions 64 et 62 comportent chacune un côté qui est sensiblement parallèle à l'axe longitudinal du capuchon et qui est référencé 66 et un côté incliné 68. On comprend que, après section des portions sécables, les parties 62 et 64 de ces portions sécables pourront être mises en contact par leur bord rectilignes 66 pour permettre un dévissage de la pièce de fixation 44 par rapport au corps de seringue, le capuchon ou plus précisément la partie formant enveloppe du capuchon servant de clé de dévissage de la pièce de fixation 44.

On va maintenant expliquer le mode d'utilisation du capuchon objet de l'invention. Dans un premier temps, l'ensemble aiguille 22 est mis en place dans le capuchon 40 de telle manière que la partie 26a de l'embase 26 de l'ensemble aiguille soit engagée dans l'orifice axial 56 de la pièce de fixation 44 assurant une liaison temporaire entre le capuchon et l'ensemble aiguille. Les extensions 28 et 30 de l'ensemble aiguille sont disposées à l'extérieur de la pièce de fixation 44. Cet ensemble qui peut être tenu par les éléments de préhension 48 et 50 est engagé sur l'extrémité du corps de seringue 12. La partie tronconique 16 du corps de seringue pénètre dans le passage axial de l'embase 26 de l'ensemble aiguille. Dans un premier temps, les extensions 28 et 30 de l'embase de l'ensemble aiguille coopèrent avec le taraudage 20 de la virole 18 du corps de seringue. Dans une phase ultérieure, le filetage 54 de la pièce de fixation 44 du capuchon vient également en prise avec le taraudage 20. L'utilisateur continue à provoquer la rotation du capuchon par rapport au corps de seringue. Ensuite, l'embase 26 de l'ensemble aiguille est bloquée en translation par la partie tronconique 16. La poursuite de la rotation du capuchon provoque un couple de serrage de la pièce de fixation 44 dans le taraudage 20. Lorsque le couple de serrage prédéterminé est atteint, les portions sécables 46 sont brisées et la partie formant enveloppe est séparée de la pièce de fixation. Cette partie 42 peut être enlevée. On comprend que la pièce de fixation 44 qui est vissée dans le taraudage 20 constitue un contre-écrou pour les extensions 28 et 30 de l'embase 26 de l'ensemble aiguille 22. L'aiguille 24 est ainsi solidement et efficacement fixée à l'extrémité du corps de seringue 12.

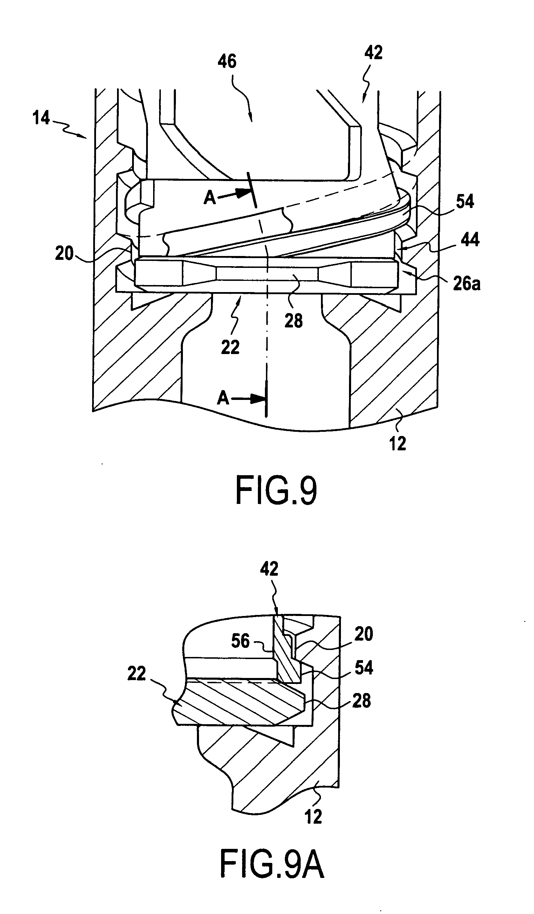

Les figures 9 et 9 A montrent en détails la solidarisation de l'aiguille 24 par la pièce de fixation 44 du capuchon sur le corps de seringue. A la fin de l'opération de vissage, la forme en triangle de l'extrémité d'attaque 54a du filetage 54 de la pièce de fixation 44 vient se coincer, par un effet de coin entre le taraudage 20 du corps de seringue et une extension radiale 28 de l'embase 26 de l'aiguille. On obtient ainsi une fixation très efficace de l'aiguille sur le corps de seringue.

Lorsqu'on veut procéder au démontage de l'aiguille par rapport au corps de seringue, on utilise la partie formant enveloppe 42. Plus précisément, on engage les portions 64 des portions sécables qui sont restées solidaires de la partie formant enveloppe entre les portions 62 qui sont restées solidaires de la pièce de fixation 44. En faisant tourner la partie formant enveloppe, les faces rectilignes 66 des portions respectives viennent au contact en rotation. En outre, dans cette position relative de la pièce de fixation et de la partie formant enveloppe, les ailettes 32 et 34 de l'embase de l'aiguille viennent en coopération avec les nervures 60 et 62 ménagées à l'intérieur de la partie formant enveloppe 42. La rotation de la partie formant enveloppe entraîne donc simultanément la rotation de la pièce de fixation 44 et celle de l'embase 26 de l'ensemble aiguille. On obtient ainsi un démontage aisé de l'aiguille par rapport au corps de seringue 12.

## Revendications

1. Capuchon (40) pour aiguille (24) de seringue destinée à être montée sur le corps (12) d'une seringue (10), ledit capuchon **se caractérisant en ce qu'**il comprend :
- une partie formant enveloppe (42) pour recevoir l'aiguille ;
- une pièce de fixation (44) apte à recevoir l'embase (26) de l'aiguille et comportant sur sa face externe un filetage apte à coopérer avec un taraudage (20) ménagé à une extrémité du corps de seringue ; et
- des portions sécables (46a, 46b) pour relier ladite partie formant enveloppe et ladite pièce de fixation, les portions sécables ayant des dimensions déterminées pour qu'elles se brisent lorsqu'un couple prédéterminé est appliqué à la partie formant enveloppe alors que le filetage de la pièce de fixation a été engagé avec le taraudage du corps de seringue.

2. Capuchon selon la revendication 1, **caractérisé en ce que** ladite pièce de fixation présente un évidement central (56) apte à coopérer selon l'axe longitudinal du capuchon avec la face externe de l'embase (26) de l'aiguille.

3. Capuchon selon l'une quelconque des revendications 1 et 2, **caractérisé en ce que** la partie formant enveloppe, la pièce de fixation et les parties sécables, sont formées dans un même composant, les portions sécables étant constituées par des zones de dimensions réduites limitées par des orifices (52) réalisés dans ledit composant.

4. Capuchon selon la revendication 3, **caractérisé en ce que** lesdits orifices (52) ont des contours (66, 68) tels qu'après section des portions sécables, la partie (62) des portions sécables restant liée à la pièce de fixation forment des saillies aptes à coopérer en rotation autour de l'axe longitudinal du capuchon avec la partie (64) des portions sécables restant liées à la partie formant enveloppe également en saillie pour permettre le dévissage de la portion de fixation par rapport au corps de la seringue.

5. Capuchon selon la revendication 4, **caractérisé en ce que** la face interne de la partie formant enveloppe (42) comporte des reliefs (60, 62) aptes à coopérer en rotation avec des reliefs (32, 34) ménagés dans l'embase (26) de l'aiguille, lorsque les parties en saillie des portions sécables restant liées à la pièce de fixation et à la partie formant enveloppe coopèrent en rotation.

6. Seringue (10) comprenant :
- un corps (12) de seringue présentant une extrémité de fixation munie d'un taraudage (20);
- un ensemble aiguille (22) comprenant une aiguille (24) et une embase (26), ladite embase comportant deux extensions radiales (28, 30) aptes à coopérer avec ledit taraudage du corps de seringue ; et
- un capuchon d'aiguille (40);
ladite seringue **se caractérisant en ce que** ledit capuchon d'aiguille comprend :
- une partie formant enveloppe (42) pour recevoir l'aiguille proprement dite ;
- une pièce de fixation (44) apte à recevoir l'embase de l'aiguille et comportant sur sa face externe un filetage apte à coopérer avec le taraudage (20) ménagé à l'extrémité du corps de seringue ; et
- des portions sécables (46a, 46b) pour relier ladite partie formant enveloppe et ladite pièce de fixation, les portions sécables ayant des dimensions déterminées pour qu'elles se brisent lorsqu'un couple prédéterminé est appliqué à la partie formant enveloppe alors que le filetage de la pièce de fixation a été engagé avec le taraudage du corps de seringue, la pièce de fixation formant alors dans le taraudage du corps de seringue un contre-écrou pour les extensions radiales (28, 30) de l'embase (26) de l'ensemble aiguille (22) également engagées dans le taraudage.

7. Seringue selon la revendication 6, **caractérisée en ce que** ladite pièce de fixation présente un évidement central (56) apte à coopérer selon l'axe longitudinal avec la face externe de l'embase de l'aiguille.

8. Seringue selon l'une quelconque des revendications 6 et 7, **caractérisée en ce que** la partie formant enveloppe (42), la pièce de fixation (44) et les portions sécables (46a, 46b), sont formées dans une même composant, les portions sécables étant constituées par des zones de dimensions réduites limitées par des orifices (52) réalisés dans ledit composant.

9. Seringue selon la revendication 8, **caractérisée en ce que** lesdits orifices (52) ont des contours (66, 68) tels qu'après section des portions sécables, la partie (62) des portions sécables restant liée à la pièce de fixation forme des saillies aptes à coopérer en rotation autour de l'axe longitudinal du capuchon avec la partie (64) des portions sécables restant liées à la partie formant enveloppe également en saillie pour permettre le dévissage de la portion de fixation par rapport au corps de la seringue.

10. Seringue selon la revendication 9, **caractérisée en ce que** la face interne de la partie formant enveloppe (42) comporte des reliefs (60, 62) aptes à coopérer en rotation avec des reliefs (32, 34) ménagés dans l'embase (26) de l'aiguille, lorsque les parties en saillie des portions sécables restant liées à la pièce de fixation et à la partie formant enveloppe coopèrent en rotation.

11. Seringue selon l'une quelconque des revendications 6 à 10, **caractérisée en ce que**, lorsque l'ensemble aiguille (22) est engagé dans ledit capuchon (40), la portion (26a) de l'embase de l'aiguille portant les extensions radiales (28, 30) fait saillie hors de la pièce de fixation (44).

12. Seringue selon l'une quelconque des revendication 6 à 11, **caractérisée en ce que** l'extrémité d'attaque du filetage de la pièce de fixation produit un effet de coin, en fin de vissage, entre le taraudage (20) du corps de seringue et une extension radiale (28, 30) de l'embase de l'ensemble aiguille.

## Patentansprüche

1. Schutzkappe (40) für eine Spritzennadel (24), die dazu bestimmt ist, auf dem Körper (12) einer Spritze (10) montiert zu werden, **dadurch gekennzeichnet, dass** sie umfasst:
- einen eine Hülle (42) zur Aufnahme der Nadel bildenden Teil,
- ein Befestigungsstück (44), das geeignet ist, die Basis (26) der Nadel aufzunehmen, und umfassend auf seiner Außenseite ein Gewinde, das geeignet ist, mit einem Gewinde (20) zusammenzuwirken, das an einem Ende des Spritzenkörpers vorgesehen ist, und
- trennbare Abschnitte (46a, 46b), um den die Hülle bildenden Teil und das Befestigungsstück zu verbinden, wobei die teilbaren Abschnitte bestimmte Abmessungen haben, damit sie zerbrechen, wenn ein bestimmtes Moment an den die Hülle bildenden Teil angelegt wird, während das Gewinde des Befestigungsstücks mit dem Gewinde des Spritzenkörpers in Eingriff gelangt ist.

2. Schutzkappe nach Anspruch 1, **dadurch gekennzeichnet, dass** das Befestigungsstück eine zentrale Ausnehmung (56) aufweist, die geeignet ist, entlang der Längsachse der Schutzkappe mit der Außenseite der Basis (26) der Nadel zusammenzuwirken.

3. Schutzkappe nach einem der Ansprüche 1 und 2, **dadurch gekennzeichnet, dass** der die Hülle bildende Teil, das Befestigungsstück und die trennbaren Abschnitte in einem selben Bauteil ausgebildet sind, wobei die trennbaren Abschnitte von Zonen mit verringerten Abmessungen gebildet sind, die von Öffnungen (52) begrenzt sind, die in dem Bauteil vorgesehen sind.

4. Schutzkappe nach Anspruch 3, **dadurch gekennzeichnet, dass** die Öffnungen (52) derartige Konturen (66, 68) aufweisen, dass nach dem Abtrennen der trennbaren Abschnitte der Teil (62) der trennbaren Abschnitte, der mit dem Befestigungsstück verbunden bleibt, Vorsprünge bildet, die geeignet sind, in Drehung um die Längsachse der Schutzkappe mit dem ebenfalls vorspringenden Teil (64) der trennbaren Abschnitte zusammenzuwirken, der mit dem die Hülle bildenden Teil verbunden bleibt, um das Losschrauben des Befestigungsteils vom Körper der Spritze zu ermöglichen.

5. Schutzkappe nach Anspruch 4, **dadurch gekennzeichnet, dass** die Innenseite des die Hülle bildenden Teils (42) Reliefs (60, 62) umfasst, die geeignet sind, in Drehung mit Reliefs (32, 34), die in der Basis (26) der Nadel vorgesehen sind, zusammenzuwirken, wenn die vorspringenden Teile der trennbaren Abschnitte, die mit dem Befestigungsstück und dem die Hülle bildenden Teil verbunden bleiben, in Drehung zusammenwirken.

6. Spritze (10), umfassend:
- einen Spritzenkörper (12), der ein Befestigungsende umfasst, das mit einem Gewinde (20) versehen ist,
- eine Nadeleinheit (22), umfassend eine Nadel (24) und eine Basis (26), wobei die Basis zwei radiale Erweiterungen (28, 30) umfasst, die geeignet sind, mit dem Gewinde des Spritzenkörpers zusammenzuwirken, und
- eine Nadelschutzkappe (40),
wobei die Spritze **dadurch gekennzeichnet ist, dass** die Nadelschutzkappe umfasst:
- einen eine Hülle bildenden Teil (42) zur Aufnahme der eigentlichen Nadel,
- ein Befestigungsstück (44), das geeignet ist, die Basis der Nadel aufzunehmen, und umfassend auf seiner Außenseite ein Gewinde, das geeignet ist, mit dem Gewinde (20) zusammenzuwirken, das am Ende des Spritzenkörpers vorgesehen ist, und
- trennbare Abschnitte (46a, 46b), um den die Hülle bildenden Teil mit dem Befestigungsstück zu verbinden, wobei die trennbaren Abschnitte bestimmte Abmessungen haben, damit sie brechen, wenn ein vorbestimmtes Moment an den die Hülle bildenden Teil angelegt wird, während das Gewinde des Befestigungsstücks mit dem Gewinde des Spritzenkörpers in Eingriff gelangt ist, wobei das Befestigungsstück nun in dem Gewinde des Spritzenkörpers eine Gegenmutter für die radialen Erweiterungen (28, 30) der Basis (26) der Nadeleinheit (22) bildet, die ebenfalls in dem Gewinde in Eingriff stehen.

7. Spritze nach Anspruch 6, **dadurch gekennzeichnet, dass** das Befestigungsstück eine zentrale Ausnehmung (56) aufweist, die geeignet ist, entlang der Längsachse mit der Außenseite der Basis der Nadel zusammenzuwirken.

8. Spritze nach einem der Ansprüche 6 und 7, **dadurch gekennzeichnet, dass** der die Hülle (42) bildende Teil, das Befestigungsstück (44) und die trennbaren Abschnitte (46a, 46b) in einem selben Bauteil ausgebildet sind, wobei die trennbaren Abschnitte von Zonen mit verringerten Abmessungen gebildet sind, die von Öffnungen (52) begrenzt sind, die in dem Bauteil vorgesehen sind.

9. Spritze nach Anspruch 8, **dadurch gekennzeichnet, dass** die Öffnungen (52) derartige Konturen (66, 68) aufweisen, dass nach dem Abtrennen der trennbaren Abschnitte der Teil (62) der trennbaren Abschnitte, der mit dem Befestigungsstück verbunden bleibt, Vorsprünge bildet, die geeignet sind, in Drehung um die Längsachse der Schutzkappe mit dem ebenfalls vorspringenden Teil (64) der trennbaren Abschnitte zusammenzuwirken, der mit dem die Hülle bildenden Teil verbunden bleibt, um das Losschrauben des Befestigungsteils vom Körper der Spritze zu ermöglichen.

10. Spritze nach Anspruch 9, **dadurch gekennzeichnet, dass** die Innenseite des die Hülle bildenden Teils (42) Reliefs (60, 62) umfasst, die geeignet sind, in Drehung mit Reliefs (32, 34), die in der Basis (26) der Nadel vorgesehen sind, zusammenzuwirken, wenn die vorspringenden Teile der trennbaren Abschnitte, die mit dem Befestigungsstück und dem die Hülle bildenden Teil verbunden bleiben, in Drehung zusammenwirken.

11. Spritze nach einem der Ansprüche 6 bis 10, **dadurch gekennzeichnet, dass**, wenn die Nadeleinheit (22) in der Schutzkappe (40) in Eingriff ist, der Abschnitt (26a) der Basis der Nadel, der die radialen Erweiterungen (28, 30) trägt, aus dem Befestigungsstück (44) herausragt.

12. Spritze nach einem der Ansprüche 6 bis 11, **dadurch gekennzeichnet, dass** das Angriffsende des Gewindes des Befestigungsstücks am Ende des Schraubens einen Keileffekt zwischen dem Gewinde (20) des Spritzenkörpers und einer radialen Erweiterung (28, 30) der Basis der Nadeleinheit erzeugt.

## Claims

1. Cap (40) for a syringe needle (24) designed to be mounted on the body (12) of a syringe (10), said cap being **characterized in that** it comprises:
- a envelope-forming part (42) for receiving the needle;
- a fixation piece (44) capable of receiving the base (26) of the needle and comprising on its external surface a threaded section capable of cooperating with an internal screw thread (20) arranged at one end of the syringe body; and
- divisible portions (46a, 46b) for connecting said envelope-forming part and said fixation piece, the divisible portions having certain dimensions so that they break when a predetermined torque is applied to the envelope-forming part, when the threaded section of the fixation piece has been engaged with the internal screw thread of the syringe body.

2. Cap according to claim 1, **characterized in that** said fixation piece has a central cutout (56) capable of cooperating along the longitudinal axis of the cap with the external surface of the base (26) of the needle.

3. Cap according to any one of the claims 1 and 2, **characterized in that** the envelope-forming part, the fixation piece and the divisible parts are formed in a same component, the divisible portions being made up of zones of reduced dimensions limited by openings (52) made in said component.

4. Cap according to claim 3, **characterized in that** said openings (52) have contours (66, 68) such as after division of the divisible portions, the part (62) of the divisible portions remaining connected to the fixation piece form protrusions capable of cooperating, in rotation around the longitudinal axis of the cap, with the part (64) of the divisible portions remaining connected to the envelope-forming part also protruding for making possible the unscrewing of the fixation portion relative to the body of the syringe.

5. Cap according to claim 4, **characterized in that** the internal surface of the envelope-forming part (42) comprises reliefs capable of cooperating, in rotation, with reliefs (32, 34) arranged in the base (32, 34) of the needle, when the projecting parts of the divisible portions remaining connected to the fixation piece and to the envelope-forming part cooperate in rotation.

6. Syringe (10) comprising:
- a syringe body (12) having a fixation end provided with an internal screw thread (20);
- a needle assembly (22) comprising a needle (24) and a base (26), the said base comprising two radial extensions (28, 30) capable of cooperating with said internal screw thread of the syringe body; and
- a needle cap (40);
the said syringe being **characterized in that** said needle cap comprises:
- an envelope-forming part (42) for receiving the needle itself;
- a fixation piece (44) capable of receiving the base of the needle and comprising on its external surface a threaded section capable of cooperating with the internal screw thread (20) arranged at the end of the syringe body; and
- divisible portions (46a, 46b) for connecting said envelope-forming part and said fixation piece, the divisible portions having certain dimensions so that they break when a predetermined torque is applied to the envelope-forming part when the threaded section of the fixation piece has been engaged with the internal screw thread of the syringe body, the fixation piece thus forming in the internal screw thread of the syringe body a locknut for the radial extensions (28, 30) of the base (26) of the needle assembly (22) which are also engaged in the screw thread.

7. Syringe according to claim 6, **characterized in that** said fixation piece has a central cutout (56) capable of cooperating along the longitudinal axis with the external surface of the base of the needle.

8. Syringe according to any one of the claims 6 and 7, **characterized in that** the envelope-forming part (42), the fixation piece (44) and the divisible portions (46a, 46b) are formed in a same component, the divisible portions being made up of zones having reduced dimensions limited by openings (52) made in said component.

9. Syringe according to claim 8, **characterized in that** said openings (52) have contours (66, 68) such as after dividing the divisible portions, the part (62) of the divisible portions remaining connected to the fixation piece forms protrusions capable of cooperating in rotation around the longitudinal axis of the cap, with the part (64) of the divisible portions remaining connected to the envelope-forming part also protruding to make possible the unscrewing of the fixation portion relative to the body of the syringe.

10. Syringe according to claim 9, **characterized in that** the internal surface of the envelope-forming part (42) comprises reliefs (60, 62) capable of cooperating in rotation with reliefs (32, 34) arranged in the base (26) of the needle, when the protruding parts of the divisible portions remaining connected to the fixation piece and to the envelope-forming part cooperate in rotation.

11. Syringe according to any one of claims 6 to 10, **characterized in that**, when the needle assembly (22) is engaged in said cap (40), the portion (26a) of the base of the needle having the radial extensions (28, 30) protrudes out of the fixation piece.

12. Syringe according to any one of claims 6 to 11, **characterized in that** the engaging end of the threaded section of the fixation piece produces a wedge effect, after screwing, between the internal screw thread (20) of the syringe body and a radial extension (28, 30) of the base of the needle assembly.
